# EUROPÄISCHE PATENTSCHRIFT

(11) **EP 4 118 039 B1**
(45) Veröffentlichungstag und Bekanntmachung des Hinweises auf die Patenterteilung: **19.02.2025**
(21) Anmeldenummer: 21711497.4
(22) Anmeldetag: 08.03.2021
(51) Int. Cl.: C02F 1/44

(54) **SYSTEM ZUR ANALYSE VON WASSER**
SYSTEM FOR ANALYSING WATER
SYSTEME POUR ANALYSER DE L'EAU

(30) Priorität: 09.03.2020 DE 102020106340
(43) Veröffentlichungstag der Anmeldung: 18.01.2023
(73) Patentinhaber: Analytik Jena GmbH+Co. KG, 07745 Jena (DE)
(72) Erfinder: LEHMANN, Roland, 07743 Jena (DE)
(74) Vertreter: Koslowski, Christine Adelheid
(86) Internationale Anmeldenummer: PCT/EP2021/055726
(87) Internationale Veröffentlichungsnummer: WO 2021/180620

(56) Entgegenhaltungen:
- US-A1- 2013 075 331
- US-A1- 2014 342 397
- US-A1- 2016 109 425
- US-A1- 2016 340 204
- US-A1- 2019 039 022
- US-A1- 2019 209 968

## Beschreibung

Die vorliegende Erfindung betrifft ein System zum Feststellen der Notwendigkeit einer Maßnahme und/oder eines Erfolgs einer Maßnahme bei Wasser, insbesondere Trinkwasser, Prozesswasser oder Abwasser, in einer Leitung. Das Wasser kann sich dabei beispielsweise in einer Rohrleitung oder in einem Behälter befinden. Im Falle eines Behälters weist der Behälter eine Zu- und/oder Ableitung zum Befüllen/Entleeren des Behälters auf.

Zur Überprüfung der Qualität von Wasser sind zahlreiche Verfahren bekannt, mit welchen sich unterschiedliche chemische, physikalische oder auch mikrobiologische Parameter der jeweiligen Proben bestimmen lassen. Je nach der untersuchten Eigenschaft erfolgt entweder zunächst eine Probennahme mit anschließender Analyse im Labor oder es wird in manchen Fällen auch eine Feldanalyse direkt vor Ort durchgeführt.

Insbesondere bei Trinkwasser müssen bereits geringste Kontaminationen früh und zuverlässig festgestellt werden. Ein besonders Problem stellen in diesem Zusammenhang bakterielle Pathogene, beispielsweise *L. pneumophila* oder *P. aeruginosa,* aber auch andere Kontaminationen des Trinkwassers durch Nanopartikel und weitere Giftstoffe dar. Viele solche Kontaminationen können mit üblichen Analysemethoden oft erst mit einer Verzögerung von mehreren Tagen nachgewiesen werden können. Zudem können geforderte Grenzwerte für bestimmte Kontaminationen - in Deutschland wird z. B. gemäß der Trinkwasserverordnung ein Grenzwert von weniger als einem E.coli auf 100ml Wasser gefordert - mit gängigen Analysegeräten, welche in der Wasseranalytik eingesetzt werden, nicht immer regulär erfasst werden. Messmethoden, die dazu in der Lage sind, wie beispielsweise eine kontinuierliche PCR-Messung, sind dagegen wirtschaftlich teuer und aufwendig.

Ein weiteres Beispiel für die Grenzen bisheriger Analysemethoden stellt die Ermittlung des TOC Wertes (total organic carbon) dar, welche oftmals zu undifferenziert ist, um eine verlässliche Aussage zur biologischen Qualität des Wassers zu ermöglichen. Bisherige Verfahren anhand biologischer Tests sind aufwendig und erlauben eine oftmals um Tage verzögerte Kontrolle des Trinkwassers.

Auch die zunehmende Verschmutzung der Umwelt mit industriell hergestellten Nano-Partikeln ist problematisch. Diese gelangen vermehrt in die Umwelt und somit auch in Abwässer und Trinkwasser, wo sie von Menschen und Tieren aufgenommen werden. Nano-Partikel sind genau wie auch Pathogene sehr vielfältig in ihrem Aufbau und ihrer Zusammensetzung, wovon auch ihre jeweilige pathogene Wirkung abhängt. Es ist somit von zunehmender Dringlichkeit, den Anteil und die Zusammensetzung von gering konzentrierten partikulären Substanzen im Wasser zu erfassen.

Anordnungen, welche der Filterung von Flüssigkeiten - z. B. Wasser - und der Überprüfung der Filterung dienen, sind z. B. offenbart in der US 2013/075331 A1, der US 2019/209968 A1, der US 2016/340204 A1 oder der US 2019/039022 A1. Der US 2014/342397 A1 lässt sich entnehmen, wie aus einem Filter mögliche Parasiten entfernt und das so entnommene Retentat konzentriert und untersucht wird. Die Analyse eines Rententats, um Aussagen über das Filtersystem zu erhalten, beschreibt die US 2016/0109425 A1.

Eine kontinuierliche und zeitnahe Überwachung dieser vielfältigen Parameter scheitert derzeit daran, dass die verfügbaren Mess- und Analyseverfahren zeit- und arbeitsaufwendig sind oder in manchen Fällen eine zuverlässige Analytik fehlt. Somit liegt der vorliegenden Erfindung die Aufgabe zugrunde, eine Möglichkeit zur zuverlässigen und umfassenden Analyse bzw. Untersuchung von Wasser anzugeben, welche universell einsetzbar ist und bereits geringe Mengen an Kontaminationen detektieren kann.

Diese Aufgabe wird gelöst durch das System nach Anspruch 1.

Wasserfilter werden vielfach im Bereich von wasserführenden Leitungen verbaut, dienen der Filterung bestimmter Substanzen aus dem Wasser und bewirken eine Zurückhaltung von Partikeln entsprechend einer vorgegebenen Porengröße. Als Retentat bzw. Filterkuchen wird im Zusammenhang mit Filtern die Substanz bezeichnet, welche bei dem durch den Filter bewirkten Trennprozess zurückgehalten wird. Das Retentat von Wasserfiltern wird üblicherweise verworfen bzw. entsorgt. In diesem Retentat liegen normalerweise partikuläre Kontaminationen (Mikroorganismen, Nanopartikel etc.) des Wassers jedoch aufkonzentriert vor. Demzufolge eignet sich das Retentat bestens für eine quantitative und qualitative Analyse, insbesondere können auch gering konzentrierte Kontaminationen anhand des Retentats nachgewiesen werden. Eine Untersuchung des Retentats, welches in großen Mengen in bestehenden öffentlichen und privaten Infrastrukturen verfügbar ist, bietet folglich ein bislang ungenutztes, erhebliches Informationspotential.

Die vorliegende Erfindung ermöglicht vorteilhaft, partikuläre Kontaminationen, welche in den Retentaten vorhanden sind, umfassend, dezentral und zeitnah festzustellen.

In einer Ausgestaltung wird zum Analysieren des Retentats eine Mess-/Analyseeinrichtung, insbesondere ein Mess-/Analysegerät zur Analyse zumindest eines gelösten und partikulären Stoffes, vorzugsweise ein Massenspektrometer, ein Atomabsorptionsspektrometer ein Atomemissionsspektrometer, ein Raman-Spektrometer und/oder ein hyperspektrales Mikroskop, insbesondere im Bereich von sichtbarem Licht oder im nahen Infrarotbereich, verwendet. Die Kombination und/oder Verwendung verschiedener Mess-/Analyseeinrichtungen stellt sicher, dass die vielfältigen möglichen Kontaminationen alle nachgewiesen werden können. Es können jeweils ein oder mehrere unterschiedliche Mess-/Analyseeinrichtungen für die durchzuführende Analyse zeitgleich oder nacheinander durchgeführt werden. Dabei erfolgt die gezielte Auswahl oder Kombination der unterschiedlichen Methoden je nach zu erwartenden Informationen. Die Massenspektrometrie eignet sich insbesondere zur Feststellung der elementaren Zusammensetzung, die Raman-Spektroskopie liefert beispielsweise Informationen zur molekularen Zusammensetzung der jeweiligen Probe und zur phänotypischen Bestimmung von Bakterien. Eine hyperspektrale Mikroskopie im VIS und NIR-Bereich wiederum kann Aufschluss über chemisch-strukturelle sowie physikalische Informationen geben, und auch eine phänotypische Bestimmung von Bakterien ermöglichen. Durch eine Kombination verschiedener Nachweistechnologien werden verschiedenste Informationen der vermessenen partikulären Kontaminationen erfasst. Diese Informationen, sowohl zur elementaren und molekularen Zusammensetzung der Partikel als auch zur Struktur und Form, ermöglichen in ihrer Kombination eine bessere Identifikation der jeweiligen partikulären Kontaminationen. Es sei darauf verwiesen, dass die hier beispielhaft angegebenen Mess-/Analyseeinrichtungen und deren jeweilige Möglichkeiten keineswegs abschließend sind. Vielmehr kann je nach Anwendung, beispielswiese je nach festzustellender Kontamination, jeweils ein geeignetes Verfahren gewählt werden.

Es ist von Vorteil, wenn die Analyse im Single-Particle- oder im Single-Cell-Modus durchgeführt wird. In einem derartigen Betriebsmodus wird anstelle eines kontinuierlichen Signals jeweils ein kurzes, starkes Signal von einem definierten Partikel oder einer definierten Zelle empfangen. Beispiele für Vorrichtungen und Verfahren dieser Betriebsmoden in der Spektroskopie sind beispielsweise in der US 7479,630 B2 beschrieben.

Bei den Wasserfiltern handelt es sich jeweils um einen selbstreinigenden Filter. Selbstreinigende Filter sind an sich aus dem Stand der Technik bekannt und zeichnen sich dadurch aus, dass sie in hohem Durchsatz große Volumina an Wasser filtern und Partikel bis zu einem Durchmesser von 0,2 µm und kleiner zurückhalten können.

Zur Reinigung werden die Filter regelmäßig gespült. Dabei werden die auf dem Filter gebundene Partikel wieder vom Filter gelöst. So wird in einer vorteilhaften Ausgestaltung das Retentat während eines Reinigungsprozesses des Wasserfilters entnommen. Beispielsweise kann das zur Reinigung des Filters verwendete Spülwasser, welches das Retentat enthält, dem jeweiligen Filter entnommen werden.

Durch geeignete Wahl des jeweiligen Filters kann darüber hinaus die Partikelgröße des im Spülwasser enthaltenen Filtrats geeignet eingeschränkt werden. Dies erlaubt beispielsweise eine Filtrierung bis hin zu einer Partikelgröße von 0,2 µm, welche direkt im Größenbereich bakterieller Pathogene liegt. Mittels Vorfilterung kann somit das finale Filtrat in diesem Fall auf einen Größenbereich von beispielsweise 0,2 bis 10 µm eingeschränkt und als Spülwasser der Analyse zugeführt werden

Da die jeweiligen Kontaminationen in dem Retentat von einem Filter bereits aufkonzentriert vorliegen, kann das Retentat in vielen Anwendungsfällen direkt ohne weitere Vorbereitungsschritte analysiert werden. Es ist aber gemäß einer Ausgestaltung des Verfahrens ebenfalls möglich, dass das Retentat zur Vorbereitung der Analyse vorab kultiviert wird.

Vorteilhaft lassen sich mittels einer massenspektrometrischen Analyse, insbesondere mittels eines Massenspektrometers mit induktiv gekoppeltem Plasma pathogene Bakterien anhand der quantitativen Analyse ihrer elementaren Zusammensetzung identifizieren und differenzieren.

Eine Ausgestaltung beinhaltet, dass das Wasser auf das Vorliegen einer partikulären Kontamination, insbesondere in Form von Mikroorganismen und/oder Nanopartikeln, untersucht wird.

Eine weitere Ausgestaltung beinhaltet, dass das Wasser auf das Vorhandensein, die Beschaffenheit und/oder die Umgebung von Bakterien und/oder Pathogenen, insbesondere eine Konzentration und/oder räumliche Verteilung der Bakterien, untersucht wird.

Noch eine Ausgestaltung sieht vor, dass eine Gesamtkeimzahl im Wasser ermittelt wird.

Schließlich beinhaltet eine Ausgestaltung, dass das Wasser auf das Vorhandensein von Noxen, beispielsweise Blei oder Arsen, untersucht wird.

Die hier genannten Beispiele für unterschiedliche Kontaminationen, welche im Rahmen des erfindungsgemäßen Systems erfassbar sind, sind jedoch keineswegs abschließend. Vielmehr können zahlreiche weitere Eigenschaften von Wasser in Abhängigkeit der jeweils verwendeten Mess- und Analysemethoden untersucht werden.

Bei der Leitung des Systems kann es sich beispielsweise um eine Rohrleitung handeln, in welcher sich die Wasserfilter befinden. Das Wasser kann sich aber ebenfalls in einem stehenden Reservoir befinden, beispielsweise in einem Behälter. In letzterem Falle ist es von Vorteil, wenn das Behältnis über eine Zu- und/oder Ableitung verfügt, in welcher die Wasserfilter angeordnet sind. Die Einrichtung zum Bereitstellen des Retentats umfasst die Wasserfilter, welche wiederum insbesondere eine Filtereinheit, beispielsweise in Form einer Membran, umfassen.

Erfindungsgemäß handelt es sich bei den Wasserfiltern um selbstreinigende Filter, wobei die Einrichtung Mittel zur Umkehrung einer Flussrichtung des Wassers durch die Wasserfilter in Form der Saugscanner umfasst.

Die Erfindung wird anhand der nachfolgenden Figuren näher erläutert. Es zeigt:
Fig.1 eine erste Ausgestaltung für ein nicht erfindungsgemäßes System zum Feststellen der Notwendigkeit einer Maßnahme und/oder eines Erfolgs einer Maßnahme bei Wasser in einer Leitung mit einem lösbar in eine Rohrleitung integrierten Wasserfilter,
Fig. 2 eine zweite Ausgestaltung für ein nicht erfindungsgemäßes System mit einem selbstreinigenden Filtern mit Mittel zur Umkehrung der Flussrichtung des Mediums durch den Filter;
Fig. 3 eine dritte Ausgestaltung für ein nicht erfindungsgemäßes System mit einem selbstreinigenden Filter und einem Saugscanner, und
Fig.4 eine Ausgestaltung für ein erfindungsgemäßes System mit zwei Wasserfiltern und zwei integrierten Mess-/Analyseeinrichtungen.

In den Figuren sind gleiche Elemente mit demselben Bezugszeichen versehen.

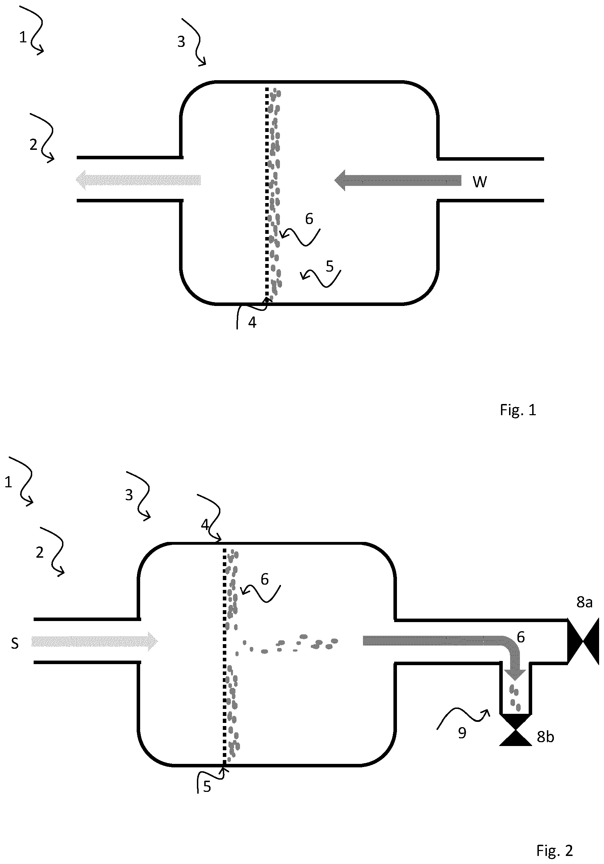

Fig. 1 zeigt eine erste Ausgestaltung eines nicht erfindungsgemäßen Systems 1. Dargestellt ist eine Rohrleitung 2 mit einer Einrichtung 3 zum Bereitstellen des Retentats von dem Wasserfilter 4, bei welchem eine Filtereinheit 5 lösbar in die Rohrleitung 2 integriert ist. Das Wasser W fließt in der durch die beiden Pfeile angegebenen Flussrichtung durch den Filter 4. Dabei sammelt sich das Retentat 6 an der Filtereinheit 5, welches durch den Filter 4 zurückgehalten wird. Bei einem Ausbau der Filtereinheit 5 aus der Rohrleitung 2 kann das Retentat 6 entnommen werden und mit einer hier nicht dargestellten Mess-/Analyseeinrichtung 7 analysiert werden. Bei der in Fig. 1 dargestellten System 1 sind die Einrichtung 3 und die Mess-/Analyseeinrichtung getrennt voneinander angeordnet. In anderen Ausgestaltungen kann das System aber auch in einer kompakteren Bauweise ausgebildet sein, derart dass die Mess-/Analyseeinrichtung 7 gemeinsam mit der Einrichtung 3 angeordnet ist.

Bei dem in Fig. 2 dargestellten System 1 handelt es sich um ein System 1 mit einem selbstreinigenden Filter 4 in Form eines Rückspülfilters. Im Normalbetrieb wird die Rohrleitung 2 von Wasser W durchströmt ähnlich wie in Fig. 1. In Fig. 2 ist nun ein Reinigungsprozess des Filters 4 illustriert. Die Einrichtung 3 umfasst zwei Ventile 8a,8b. Bei dem ersten Ventil 8a handelt es sich um ein Umschaltventil, mittels welchem die Strömungsrichtung des Wassers umgekehrt werden kann. Während des Reinigungsprozesses durchströmt demnach Spülwasser S die Rohrleitung 2 in der durch die Pfeile gekennzeichneten Flussrichtung. Während des Reinigungsprozesses wird ferner das zweite Ventil 8b geöffnet, sodass das das Retentat 6 enthaltende Spülwasser S in die zusätzliche Leitung 9 gelangt, durch welche es beispielsweise direkt eine wieder nicht dargestellten Mess-/Analyseeinrichtung zugeführt werden kann. Entweder die Mess-/Analyseeinrichtung 7 ist direkt an die Leitung 9 angeschlossen, oder das Retentat 6 enthaltene Spülwasser S kann mittels der Leitung 9 entnommen und eine separat von der Einrichtung 3 angeordneten Mess-/Analyseeinrichtung zugeführt werden.

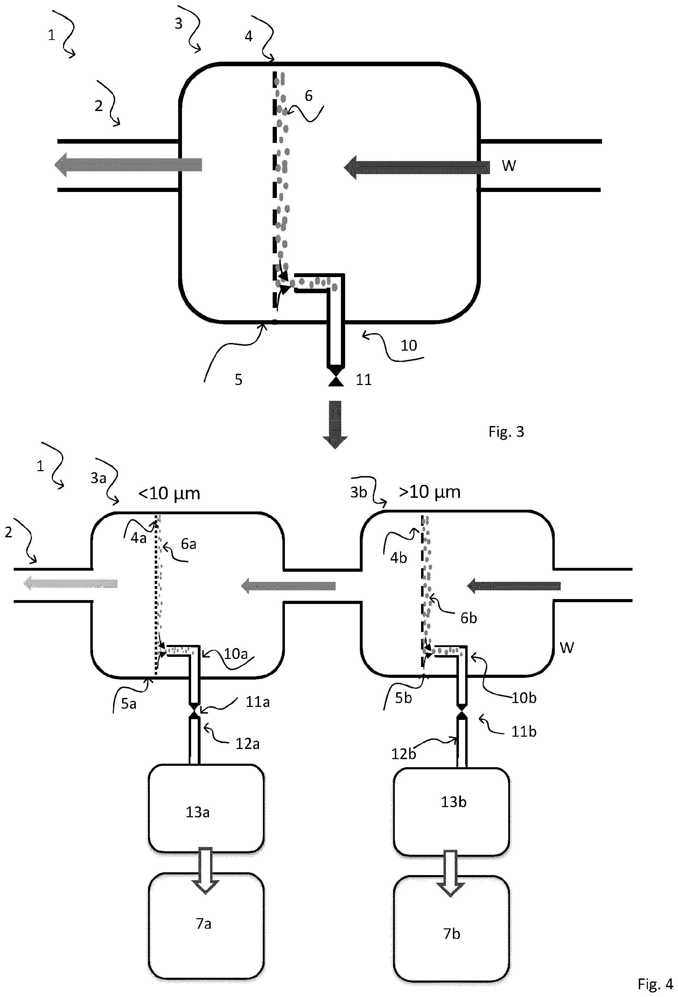

Eine Ausgestaltung eines nicht erfindungsgemäßen Systems 1 mit einem selbstreinigenden Filter 4 ist Gegenstand von Fig. 3. Hierbei handelt es sich um einen Filter 4 mit einem Saugscanner 10, mittels welchem das Retentat 6 von der Filtereinheit 5 während eines Reinigungsprozesses entfernt werden kann. Der Saugscanner 10 weist ein Ventil 11 auf, über welches das Retentat 6 entnommen werden kann.

Eine Ausgestaltung für ein erfindungsgemäßes System 1 ist Gegenstand von Fig. 4. Das System 1 umfasst zwei Einrichtungen 3a,3b zur Bereitstellung jeweils eines Retentats 6a,6b mit zwei unterschiedlichen Filtern 4a,4b. Der erste Filter 4 dient zur Filterung größerer Partikel im Wasser, der zweite Filter 4b zur Filterung kleinerer Partikel. Die beiden verwendeten Filtereinheiten 5a,5b weisen demnach unterschiedliche Porengrößen auf, wobei die Porengröße der ersten Filtereinheit 5a größer ist als die Porengröße der zweiten Filtereinheit 5b. Für die hier gezeigte Ausgestaltung sei angenommen, dass die erste Filtereinheit 5a dazu ausgebildet ist, Partikel mit Durchmessern >10 µm und die zwei Filtereinheit 5b Partikel mit Durchmessern < 10 µm zu filtern.

Bei beiden Filtern 4a, 4b handelt es sich um selbstreinigende Filter mit jeweils einem Saugscanner 10a, 10b, welcher jeweils über ein Ventil 11a,11b und eine Zuleitung 12a,12b mit einer Einheit 13a, 13b zur Aufbereitung der Probe und einer Mess-/Analyseeinrichtung 7a,7b verbunden ist. Die Einheiten 13a, 13b zur Probenaufbereitung sind jeweils optional. Beispielsweise können diese zur Kultivierung einer Probe des Retentats 6 dienen. Bei den beiden Mess-/Analyseeinrichtungen 7 kann es sich um gleiche oder unterschiedliche Mess- /Analysegeräte handeln. Durch geeignete Wahl der Filtereinheiten 5a,5b kann das Retentat 6 gezielt beeinflusst werden, indem die Partikelgröße geeignet eingeschränkt wird.

### Bezugszeichen

- 1: System
- 2: Rohrleitung
- 3,3a,3b: Einrichtung zur Bereitstellung eines Retentats
- 4,4a,4b: Wasserfilter
- 5,5a,5b: Filtereinheit
- 6,6a,6b: Retentat
- 7,7a,7b: Mess-/Analyseeinrichtung
- 8a,8b: Ventile
- 9,: Leitung
- 10a,10b: Saugscanner
- 11a,11b: Ventil
- 12a,12b: Leitung
- 13a,13b: Einrichtungen zur Probenvorbereitung

- W: Wasser
- S: Spülwasser

## Patentansprüche

1. System (1) zum Feststellen der Notwendigkeit einer Maßnahme und/oder eines Erfolgs einer Maßnahme bei Wasser (W),
umfassend
eine Einrichtung zum Bereitstellen des Retentats (6), welches ein Filterkuchen ist, von einem ersten Wasserfilter (4a) und einem zweiten Wasserfilter (4b) in einer Leitung (2); und
zwei Mess-/Analyseeinrichtungen (7a, 7b) zur Untersuchung und/oder Analyse des Retentats (6) bezüglich zumindest einer Eigenschaft oder seiner chemischen Zusammensetzung,
wobei der erste Wasserfilter (4a) zur Filterung größerer Partikel und der zweite Wasserfilter (4b) zur Filterung kleinerer Partikel im Wasser (W) dient,
wobei der erste Wasserfilter (4a) und der zweite Wasserfilter (4b) jeweils ein selbstreinigender Filter mit jeweils einem Saugscanner (10a, 10b) ist, und
wobei die Saugscanner (10a, 10b)jeweils über ein Ventil (11a, 11b) und eine Zuleitung (12a, 12b) mit einer Mess-/Analyseeinrichtung (7a, 7b) verbunden sind.

## Claims

1. System (1) for determining the necessity of an action and/or a success of an action on water (W), comprising means for providing the retentate (6), which is a filter cake, from a first water filter (4a) and a second water filter (4b) in a conduit (2); and two measuring/analyzing means (7a, 7b) for examining and/or analyzing the retentate (6) with respect to at least one property or its chemical composition,
wherein the first water filter (4a) is used for filtering larger particles and the second water filter (4b) is used for filtering smaller particles in the water (W),
wherein the first water filter (4a) and the second water filter (4b) are each a self-cleaning filter with a respective suction scanner (10a, 10b), and
wherein the suction scanners (10a, 10b) are each connected to a measuring/analyzing device (7a, 7b) via a valve (11a, 11b) and a supply line (12a, 12b).

## Revendications

1. Système (1) pour déterminer la nécessité d'une action et/ou le succès d'une action sur l'eau (W), comprenant des moyens pour fournir le rétentat (6), qui est un gâteau de filtre, à partir d'un premier filtre à eau (4a) et d'un second filtre à eau (4b) dans un conduit (2) ; et deux moyens de mesure/analyse (7a, 7b) pour examiner et/ou analyser le rétentat (6) en ce qui concerne au moins une propriété ou sa composition chimique,
dans lequel le premier filtre à eau (4a) est utilisé pour filtrer les plus grosses particules et le second filtre à eau (4b) est utilisé pour filtrer les plus petites particules dans l'eau (W),
dans lequel le premier filtre à eau (4a) et le second filtre à eau (4b) sont chacun un filtre autonettoyant avec un scanner d'aspiration respectif (10a, 10b), et
dans lequel les scanners d'aspiration (10a, 10b) sont chacun reliés à un dispositif de mesure/analyse (7a, 7b) via une vanne (11a, 11b) et une conduite d'alimentation (12a, 12b).
